# EUROPEAN PATENT APPLICATION

(11) **EP 3 100 995 A1**
(43) Date of publication of application: **07.12.2016**
(21) Application number: 15742992.9
(22) Date of filing: 28.01.2015
(51) Int. Cl.: C07C 6/12, C07C 2/66, C07C 15/107, B01J 29/08

(54) **METHOD FOR PRODUCING SYNTHETIC ALKYL AROMATIC LUBRICANT OIL FOR REFRIGERATION COMPRESSORS BY SELECTION OF SOLID ACID CATALYSTS MODIFIED DURING THE INTEGRATED ALKYLATION AND TRANSALKYLATION PROCESS IN REACTIVE DISTILLATION COLUMNS**

(30) Priority: 28.01.2014 BR 1402154
(71) Applicant: Whirlpool S.A., 05425-070 São Paulo - SP (BR)
(72) Inventor: ARANDA, Donato Alexandre Gomes, 22795-080 Rio de Janeiro (BR); DOS SANTOS, Expedito Raimundo Pereira, 13076-061 Campinas (BR); MACHADO, Rosângela Maria, 89218-550 Joinville (BR)
(74) Representative: Soldatini, Andrea
(86) International application number: PCT/BR2015/050005
(87) International publication number: WO 2015/113129

(57) **Abstract**

The present invention refers to a process of obtaining alkylaromatic compounds where there is a higher selectivity for the production of monoalkylaromatic compounds by selection of modified acid solid catalysts in an integrated process comprising an alkylation and transalkylation of aromatic and/or polyaromatic compounds for the selective production of monoalkylaromatic compounds, as well as regeneration (in situ) of acid solid catalysts selected for this process. The process of the present invention enables the achievement of monoalkylated compounds with high rates of conversion, selectivity and yield.

## Description

### Field of the Invention

The present invention refers to a process for obtaining alkylaromatic compounds, such as mono-, di- and polyalkylaromatics, by heterogenous alkylation reaction of aromatic compounds, such as benzene, toluene, ethylbenzene and cumene, with olefins, as well as by transalkylation of derivative polyalkylaromatic compounds, in the presence of modified acid solid catalysts in reactive catalytic distillation columns in this process. In one embodiment, the alkylaromatic compounds are for application as lubricating oils with improved lubricity and enhanced performance coefficient for cooling machines, such as hermetic compressors of the type used in cooling systems.

### Background art

The change of refrigerating fluids into cooling systems of the compounds based on chlorofluorocarbons (CFCs), not environmentally friendly, to the hydrofluorocarbons (HFCs) compounds and, later on, for refrigerating fluids based on hydrocarbons (HC), required a change in the lubricating oils used in these cooling systems, according to the need of physicochemical adaptation between the refrigerant fluid and lubricating oil in compressors in said cooling systems, in order to avoid, for example, reactions producing acids and other harmful components to the integrity and efficiency of such systems.

The use of alkylaromatic compounds as lubricating oil in these cooling systems, has required the development of such oils with physicochemical characteristics more specific and differentiated for application in cooling machines, like the high efficiency hermetic compressors, demonstrating a great solubility in the refrigerant fluid, suitable viscosities in temperatures of operation with excellent lubricity, low mechanical resistance with low friction coefficient, a wide range of temperature for operation, high thermal stability, be hydrolytically stable, low foaming, among others properties. (Table 1)

**Table 1**

| Specifications of Alkylaromatic Lubricating Oil | | |
|---|---|---|
| **Characteristics** | **Test Method** | **Specification** |
| Density, 20°C, g/cm³ | POGTPP 00179 | 0,845 - 0,870 |
| Viscosity, 40°C, cSt | DIN 53015 | 4,10-4,80 |
| Viscosity, 100°C, cSt | DIN 53015 | 1,25 - 1,60 |
| Miscible Test, HFC134a | DIN 51351 | Max. -50 |
| Flash Point, °C | DIN EN ISO 2592 | Min. 135 |
| Combustion Point, °C | DIN EN ISO 2592 | Min. 145 |
| Dielectric Strength, KV | ASTM D877 | Min, 30 |
| TAN, mgKOH/g | ASTM D974 | Max. 0,03 |
| Humidity, ppm | ASTM E1064 | Max. 100 |
| Color | ASTM D1500 | Max. 1,0 |
| Infrared Spectrometry Analysis (FTIR) | POGTPP00450 | Linear alkylbenzene + 2 ± 0,5% mass BTP |
| Gas Chromatography with Mass Spectrometry (GC/MS) | POGTPP00222 | Linear alkylbenzene + 2 ± 0,5% mass BTP |

The permanent search for lubricating oils with these improved characteristics, technically and economically enables the continuous development of new cooling systems, sheltering the limits of mechanical solutions required in the production of such systems, supporting the permanent challenges in the lowering of energy consumption, noise level, vibration level, operational reliability with great autonomy and own consumption of general materials.

For cooling systems, solutions are known in the art using the alkylbenzene lubricating oil (WO2007/003024).

The document (Cooper, W. D.; Downing R. C.; and Gray, J. B, "Alkyl Benzene as a Compressor Lubricant" (1974) International Compressor Engineering Conference - School of Mechanical Engineering - Freon Products Division) presents the advantages of using the alkylaromatics as lubricating oils in comparison with paraffinic and naphthenic mineral oils.

The industrial manufacture of alkylaromatic compounds, produced in a global commercial scale using several technologies, usually occurs by the alkylation reaction of aromatic compounds with linear or branched olefins, containing from C₂ to C₃₀ carbon atoms, with greater commercial interests in the ranges of C₂ to C₃, C₄ to C₈, C₁₀ to C₁₈ and C₂₀ to C₃₀, pure or mixtures thereof. Industrially, the alkylaromatics are produced for many industrial segments, from polymeric monomers to basic petrochemicals such as ethyl and propyl benzenes, co-monomer in polyolefins, intermediate in the production of oxo alcohols, mercaptanes, epoxides, amines e oxide amines, oils, synthetic lubricants, lubricating additives, epoxides, surfactants, synthesis of fatty acids, alkylated aromatic compounds, hydrogenated oligomers, and others. The alkylation of aromatic compounds with olefins is an important industrial process being in permanent development and evolution.

Usually, in more than 90% of global industrial facilities, the process for obtaining the alkylaromatics occurs by homogeneous catalysis of Friedel-Crafts reaction by benzene alkylation with mono-olefins C₁₀ - C₁₃, having Lewis acid as catalyst, the hydrofluoric acid (HF) or aluminum trichloride (AlCl₃), mainly for the production of linear alkylbenzene (LAB-C₁₀-C₁₃) for the industrial segment of general detergent surfactants production. However, the use of these catalysts, extremely corrosive and toxic, has been questioned by care required in the maintenance of industrial equipment and mainly with the occupational and environmental safety, having a change in the new facilities for using the solid acid catalysts environmentally more friendly in these processes.

More recently, new technologies of alkylation by heterogeneous catalysis are been implemented, using a great option of acid solid catalysts in various types of reactors. A process of alkylation was developed by UOP/CEPSA, known as process UOP-Detal™ DA-114 Catalyst, described by Tanabe K. and Hölderich W.F., Appl. Catal. A: General 181 (1999) 399, among others. The alkylation reaction of benzene with the mono-olefins occurs in moderated conditions, in liquid phase, in a fixed bed reactor, using a silica-aluminum solid catalyst, (Zeolite Mordenite) containing from 1 to 3,5% of fluorides. The process Detal™ is integrated with the process Pacol™ of UOP in which linear paraffins are dehydrogenated to mono olefins, and used in the alkylation of benzene. Therefore, the new facilities for the production of alkylbenzenes in global commercial scale, follow this new technology to replace the technologies with homogeneous catalysis, either with catalysts AlCl₃, which are being deactivated all around the world, or with the catalyst HF, still being widely used as the principal technological platforms for the production of alkylbenzenes, for the surfactants production segment, despite of the drawbacks of hydrofluoric acid (HF) are extremely corrosive and toxic.

Several other technologies have been developed in the alkylation of aromatic compounds with olefins, always presenting a great commercial interest in the production of a great variety of products for the various industrial segments. It is observed a migration for the latest alkylating technologies, with the use of safer, more selective catalysts, preferably in solid state, with reactor of fixed beds containing catalysts e with reaction multi-stages, such as occurs with the recent technology UOP-Detal™ DA-114 Catalyst in the production of alkylbenzenes C₁₀-C₁₄, for the industrial segment of surfactants.

The alkylation reaction of benzene is very used and has been much studied in chemical and petrochemical industry for the production of monoalkylaromatics of great commercial interest, as well as the reaction of transalkylation of polyalkylaromatics to increase the conversion and selectivity for corresponding monoalkylbenzenes. These studies cover a large number of industrial processes, either in liquid phase or in steam phase, either by homogeneous catalysis r heterogeneous catalysis, with linear or branched olefins <C30 carbon atoms, using catalysts of different physicochemical characteristics for each need researched. The studies of homogeneous and heterogeneous catalysis in this reaction of alkylation and transalkylation have contributed significantly for its continuous development.

The patent ES 2 133 850 *"Procedimiento para la alquilación y transalquilación de aromáticos"* mentions the benzene alkylation with olefins (C₃) propene, for the production of alkylbenzene "cumene" in liquid phase, in a temperature from 100 to 130°C, with pressure of 3 to 4 bar, and with a benzene/olefins molar ration of 1:2,5 to 1:3,5. This alkylation occurs by homogeneous catalysis with many types of solutions of AlCl₃/Benzene + polyalkylated as catalyst. The polyalkylbenzenes generated as byproducts are also transalkylated with benzene, simultaneously to the alkylation reaction, using the same catalyst. This is done with the aim to increase the conversion to the desired monoalkylbenzene, the "cumene", considering the relatively low conversion rates obtained with this technique.

The patent EP 1 059 277 A1 *"Aromatic conversion process"* refers to a process of transalkylation of polyalkylbenzenes compounds with high porosity solid catalysts (Zeolites-Y) with superficial area <500m²/g. The benzene alkylation occurs in combination with the transalkylation of polyalkylated, increasing the selectivity for monoalkylbenzenes. The benzene alkylation, in steam phase, with the olefin (C₂) etene or (C₃) propene for the production of etilbenzeno and cumene respectively, with a benzene/(etene or propene) molar ratio of 18:1, temperatures of (371 to 454)°C, pressure of (18 to 32)kgf/cm², in a multistage reactor comprised of serial catalytic fixed beds, charged with the catalyst of solid alkylation, silicate of pentasyl family, like high silica molecular sieves, such as standard zeolites ZSM-5, with a ratio of silica/alumina of (220:1 a 320:1), an average pore size and a superficial area of 700 to 800 m²/g. The transalkylation of polyalkylbenzenes (from 2 to 3 carbon atoms of alkyl group), occurs in liquid phase, with benzene/polyalkylbenzene ratio of 85% mass, temperatures of (66 to 288)°C, pressure of (35 to 62)kgf/cm², in the presence of a faujasite zeolite-Y catalyst, with silica/alumina molar ratio of 2:1-5:1, of high porosity and a superficial area between (350-400)m²/g. Also, this process does not permit achieving high degrees in energy efficiency and high conversion rates, above 90%.

The patent document PCT US 99-24275 - WO 00/23405 *"Alkylation of Benzene to form Linear Alkylbenzenes Using Fluorine-Containing Mordenites"* refers to the use of a mixture of different kinds of acid solid catalysts fluoro-mordenite in the manufacture of linear alkylbenzene (LAB) by benzene alkylation with olefins with 10 to 14 carbon atoms to the production of surfactants. The mordenite-type zeolite containing fluoro is undesirably prepared by treatment with an aqueous solution of hydrogen fluoride. It also mentions that this process produces a LAB with high isomer content of 2-phenyl according to this catalyst, F-Zeolite with low sodium content <0,1%m, silica/alumina molar ratio 10:1 - 50:1, acidity <0,35 meq/g, commercially available, treated and added 0,5 - 1 % fluoro mass. It also uses another catalyst of conventional acid solid alkylation, zeolite mordenite type, without treatment with HF. The two catalysts are used in reactors of mixed fixed bed, carrying a mixture of these catalysts, or in an assembly of serial fixed bed, carried with these separated catalysts, in relative proportions, adjusted to provide a desired content of isomer of 2-phenyl in the final product. Many types of catalysts were tested, more specifically, the catalyst (A): F-LZM-8, SiO₂/Al₂O₃ 17:1, Na₂O <0,02%m, superficial area of 517 m2/g, in powder of Union Carbide Corp., dried at 120°C for 24hs e calcined at 538°C for 6h e with a second catalyst (B): F-Zeolite CBV-20A, SiO₂/Al₂O₃ 20:1; Na₂O <0,02%m, superficial area of 550 m2/g, with 1/16" extruded diameter of PQ Corp, dried at 120°C for 24hs in vacuum and calcined at 538°C for 12hs. Both catalysts produced a LAB with high concentration of 2-phenyl isomers (>70%mass), which is the main objective desired. This process uses parameters and catalysts that make it economically and environmentally undesirable.

The patent document PCT/US95/16931 - WO 96/20905, " Catalyst and Process for Alkylation of Aromatics" refers to the alkylation of aromatic non-oxygenated hydrocarbons such as benzene, toluene, xylene, and mixtures thereof with high molecular weight olefins (C₁₆ to C₂₈), preferably with alpha olefins having from 20 to 24 carbon atoms per molecule, in the presence of metal oxides acid solid catalysts such as zirconium oxide and ZrO₂.H₂SO₄ or tin oxide as SnO₂.H₂SO₄ or ZrO₂.WO₃. The catalytic studies of metal oxides showed excellent performance for this difficult alkylation of heavy olefins> C₂₀. They are good catalysts when used without exposure to humidity contained in hydrocarbons or to atmospheric humidity, which requires expensive facilities and procedures.

The patent document PCT/US89/02122 (WO 89/12613) "Improved Process for the Transalkylation of Polyalkylbenzenes" refers to a conversion improvement process in the transalkylation reaction of polyalkylbenzenes to monoalkylbenzenes in the presence of a zeolite solid catalyst and hydrogen gas. Their studies focused on improving the conversion in the transalkylation of polyalkylbenzenes resulting from the alkylation reaction of benzene with ethene (C₂) and propene (C₃) in the production of monoalkylbenzene (ethylbenzene and cumene), raw materials for the production of styrene and phenol respectively. It recommends using hydrogen gas at a H₂/Polyalkylbenzene molar ratio of 1:3, benzene / Polyalkylbenzene molar ratio of 8:1 (6:1-10: 1), temperature of 200°-550°F, pressure of 250-1000 psig. This process increases the life of the commercial catalyst typically used in this reaction, allowing using a low molar ratio of benzene /polyalkylbenzene, and reducing the frequency of regeneration and leading to a higher conversion to monoalkylbenzene. However, this process requires the use of hydrogen gas and high energy consumption to meet the required process parameters.

Also known are processes of alkylation of benzene with 1-dodecene in silicotungstic acid catalyst supported on zirconium (STA/ZrO₂) (Sawant, Dhanashri P.; and Halligudi S.B. "Alkylation of benzene with olefins over zirconia supported 12-silicotungstic acid" Journal of Molecular Catalysis A, Chemical 237 (2005) 137-145). The catalyst with 15% by weight of STA, calcined at 750°C showed higher catalytic activity at 130°C with benzene/1-dodecene molar ratio of 10:1 and catalyst concentration of 3% by weight (relative to the total reaction mixture) and 4 hours of reaction, carried out in Parr-autoclave, pressurized with N₂ (900-100) psi. The maximum conversion of 1-dodecene was 50.8%. Although the reaction is conducted at relatively low temperatures and pressure, time and the reaction conversion efficiency are unacceptably low.

Da Z., Magnoux P. and Guisnet M., Catal. Letters 61 (1999) 203 have investigated a reaction similar to LAB formation wherein toluene is alkylated with 1-dodecene. The reaction was performed in a fixed bed reactor, at 90°C, with a molar ratio of toluene/dodecene equal to 3, using a HFAU zeolite as catalyst. The dodecene is completely converted in the first 40 minutes of reaction and conversion approaches zero after 210 minutes, indicating the deactivation of the catalyst. The alkylation products are especially the monododecyltoluene (MDT) and small amounts of bidodecyltoluene (BDT) at high dodecene conversions.

Siffert (Siffert, S; Gaillard, L, Su, B L. "Alkylation of benzene by propene on a series of Beta zeolites: toward a better understanding of the mechanisms." Journal of Molecular Catalysis A: Chemical 153 (2000) 267- 279) investigated the alkylation of benzene with propene in a series of H-beta zeolites (Si/Al ratio: 10, 35 and 66), using different techniques such as FTIR, NMR C¹³ and catalytic test. The H-Beta zeolite with Si/Al ratio 10: 1 is a very active catalyst for reaction with propene. However, this way leads to a rapid oligomerization even under low temperatures. Dealuminised H-Beta zeolites are less active and deactivation due to coke formation is reduced. The high ratio of benzene/propene favors the formation of cumene and suppresses the oligomerization of propylene. Triisopropyl benzene (TIPB) may also be formed at low temperatures (below 100°C) and then transformed by transalkylation in diisopropylbenzene (DIPB) at temperatures above 130°C. The activation energy decreases with the elevation of Si/Al ratio of H-Beta zeolite. Although this technique proves to be positive in terms of alkylation reaction conditions of benzene with propene, it has the drawback of H-Beta zeolite to present a more rapid deactivation, which should be very well controlled.

In the work of Perego, 1999, two amorphous mesoporous silica-aluminas are cited (MSA and MCM-41) which were compared with the amorphous micropore silica-alumina ERS-8 and beta zeolite in the alkylation of toluene with propylene in the liquid phase. MSA and MCM-41 show alkylating activity comparable to beta zeolite and higher than the ERS-8 (micropore). Their isomerization and transalkylation activities are lower than those of beta zeolite and AlCl₃ - HCl, but similar to the SPA. MSA and MCM-41, due to its good alkylating activities and low formation of oligomeric olefins, are potential catalysts if transalkylation is not required.

In the work of Liu, 2009, the alkylation of toluene with 1-octene is referred to, in liquid phase, catalyzed by MCM-41 supported in hetero poly acid (HPA) Keggin type, as well as tungstophosphoric acid (HPW) tungstosilicic acid (HSiW) and molibdophosphoric acid (HPMo). The conversion of 1-octene was 100% and the selectivity to monoalkylated products was 99.9% after 2 hours of reaction at 120°C with HSiW/MCM-41. The presence of HPA and high dispersion of mesoporous structure of MCM-41 was confirmed. HSiW(30)/MCM-41 and HPW(30)/MCM-41 produced only monoalkylated and 1-octene conversion was nearly 100%. This process requires catalysts that reduce their convenience in economic terms.

### Modifications in Zeolites

One of the most important characteristics of the zeolites is their ability to undergo changes in the morphology of their crystalline structure and these changes may lead to very significant changes in the acidity, the adsorption capacity, thermal stability, diffusion of reactants and products in their structure in the selection of form, catalytic activity, among others. Modifying zeolites for using in catalysis have been major advances in the technical aspect on some chemical and physical properties of these materials. The strategies currently used to modify chemically and structurally zeolites to produce selective catalysts with increase and/or adjustments of the activity and selectivity have obtained excellent results.

Zeolites can be modified by methods which include: ion exchange, by encapsulation of transition metal or organometallic compounds thereof, by isomorphic doping or substitution during crystallization, by post-synthesis modification of the material by dealumination by realumination, by hydrothermal treatment, among others.

A wide variety of solid catalysts for alkylation reaction of aromatic compounds with olefins has been used industrially having one of the best options for this application in the family of zeolites, more than one hundred Zeolites have been synthesized. Zeolites can be subjected to various kinds of physico-chemical treatments with the aim to improve and/or adjust its catalytic activity, selectivity and conversion. The transformation of a zeolite synthesized as a commercial catalyst requires adjustment of the required and desired physical and chemical properties to the material (acidity, porosity, stability, catalytic activity, etc.) for the reaction of interest.

Therefore, it is clearly necessary to develop more efficient processes which present, at the same time, a higher selectivity and a higher conversion rate to the desired products.

### Summary of the invention

Therefore, it is an object of the present invention to provide a process for obtaining alkylaromatic compounds, presenting high selectivity and high conversion rate for the production of monoalkylated alkylaromatic compounds from alkylation of aromatic compounds with olefins.

Another objective of the present invention is to provide a process for obtaining synthetic lubricating oils comprising the alkylaromatic compounds with improved lubricity and increased performance coefficient produced by the process of the present invention presenting specific physico-chemical characteristics for cooling machines such as high-efficiency hermetic compressors, for example, machines operating with a refrigerant fluid consisting of at least one component of a HC group (hydrocarbon), for example, an HC-600a and/or HC-290 refrigerant.

Another objective of the present invention is to provide a selection of solid acid catalysts such as the zeolites by changing and modifying its physico-chemical properties when subjected to dealumination treatment with the increase of SiO₂/AlO₃ (SAR) molar ratio and the doping with metal cations of tin and niobium in order to improve and/or adjust its catalytic activity, selectivity and conversion, turning a synthesized zeolite into a suitable catalyst in its acidity, porosity, stability, catalytic activity for the production of monoalkylated alkylaromatic compounds from the alkylation of aromatic compounds with olefins.

Another objective of the present invention is to provide an integrated process for alkylation and transalkylation in reactive distillation column by "multi-purpose" heterogeneous catalysis using these modified catalysts to obtain alkylaromatic compounds, increasing the conversion, selectivity and a greater yield to monoalkylated alkylaromatic compounds.

Yet another object of the present invention is to provide a process of the type mentioned above and to allow, in an efficient and economical way, a continuous and permanent regeneration and recovery of the catalytic activity increasing the catalyst life cycle.

The processes of the invention result in high conversion, selectivity and yield rates, for example, for the production of monoalkylated compounds, with simultaneous transalkylation of polyalkylaromatics with simple aromatic compound for obtaining monoalkylated compounds.

The inventive concept common to all protection contexts is the optimization of procedural factors for alkylation, transalkylation of aromatic compounds with olefins in zeolites and continuous catalyst regeneration being performed in reactional conditions of pressure, temperature and time substantially lower than those of usual processes.

In a first aspect, the present invention defines a process for the production of an alkylaromatic compound comprising the steps of:
- adding at least one olefin;
- adding at least one aromatic compound;
- over a catalyst comprising zeolite with silica to alumina ratio (SAR) of at least 60:1.

In an embodiment, the catalyst comprises zeolite with SAR higher than 70:1.

In an embodiment, the present invention defines a process of production of alkylaromatic synthetic lubricating oils of formula (I) 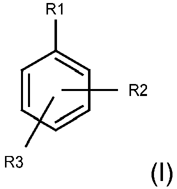 wherein
- R1 is H, CH₃, C₂H₅ or C₃H₇;
- R2 and R3 are independently H or a linear or branched alkyl radical containing from 1 to 12 carbon atoms;
comprising the steps of:
- adding at least one linear or branched, alpha or internal monounsaturated olefins containing from 1 to 12 carbon atoms;
- adding at least one aromatic compound of formula (II) 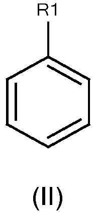 wherein
   R1 is H, CH₃, C₂H₅ or C₃H₇;
- over a catalyst comprising modified and dealuminated zeolite-HY with SAR higher than 70:1 and less than 80:1; and
wherein the molar ratio of aromatic compound to olefin is from 5:1 to 120:1.

In an embodiment, R1 is H and when R2 is H, R3 is a linear or branched alkyl having 1 to 12 carbon atoms and when R3 is H, R2 is a linear or branched having 1 to 12 carbon atoms.

In an embodiment, the total amount of carbon atoms linked to the aromatic ring of the alkylaromatic compound is from 1 to 12.

In an embodiment, over a catalyst comprising modified and dealuminated zeolite-USHY with SAR higher than 70:1 and until 80:1 for the alkylation reaction.

In an embodiment the zeolite is doped with 5% - 10% niobium and tin.

In an embodiment, the temperature of the process is maintained between 80°C and 200°C and the pressure is maintained between 100psi (7kgf/cm²) and 250psi (18kgf/cm²).

In an embodiment the catalyst has surface area between 200 and 900m²/g.

In an embodiment the catalyst is in a proportion of 5-10% by weight relative to the olefin mass (t/day) arranged in at least one catalytic bed arranged in at least one reactive catalytic distillation column.

In an embodiment the aromatic compound permeates the catalytic bed in upstream and the olefin permeates the catalytic bed in downstream.

In an embodiment the alkylaromatic compound is of formula (I) 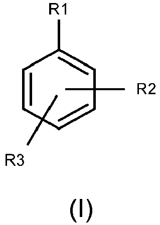 wherein
when R1 is H and R2 is H;
- R3 is a linear or branched alkyl having from 1 to 12 carbon atoms;
or when R1 is CH₃, C₂H₅ or C₃H₇,
R2 is H; and
R3 is a linear or branched alkyl having from 1 to 12 carbon atoms.

In a second aspect, the present invention defines a process for transalkylation of polyalkylaromatics for the selective monoalkylation of alkylaromatic compound comprising the steps of:
- adding at least one aromatic compound;
   wherein said aromatic compound has zero or one alkyl group linked to the aromatic compound;
- adding at least one polyalkylaromatic compound;
   wherein said polyalkylaromatic compound has at least two alkyl group linked to the polyalquilaromatic compound;
- over a catalyst comprising zeolite with SAR of at least 80:1.

In an embodiment, the product of the selective monoalkylation of the alkylaromatic compound is of formula (I) 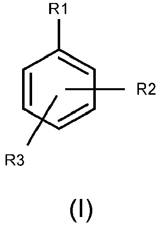 wherein when R1 is H and R2 is H, R3 is a linear or branched alkyl group having from 1 to 12 carbon atoms,
wherein the process comprises the steps of:
- adding an aromatic compound;
   wherein said aromatic compound has zero or one alkyl group linked to the aromatic compound;
- adding a polyalkylaromatic compound of formula (I) 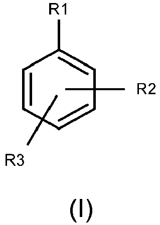 wherein R1, R2 and R3 are independently H or a linear or branched alkyl group containing from 1 to 12 carbon atoms;
   wherein said polyalkylaromatic compound has at least two alkyl groups linked to the polyalkylaromatic compound;
- over a catalyst comprising modified zeolite-HY with SAR of at least 80:1 and until 100:1,
wherein the molar ratio of the aromatic compound to the polyalkylaromatic compound is between 15:1 and 120:1.

In an embodiment, the molar ratio of the aromatic compound to the polyalkylaromatic compound is between 20:1 and 40:1.

In an embodiment, over a catalyst comprising modified and dealuminated zeolite - USHY with SAR from 80:1 to 100:1 for the transalkylation reaction.

In an embodiment the zeolite is doped with 5% - 10% niobium and tin.

In an embodiment the catalyst has surface area between 200 and 900m²/g.

In an embodiment, the aromatic compound permeates in the catalytic bed in upstream and the polyalkylaromatic compound permeates in downstream.

In an embodiment, the molar ratio of aromatic compound, such as benzene, and polyalkylaromatic compound is between 15:1 and 120: 1.

In an embodiment the temperature is between 80° C and 200° C and the pressure is maintained between 100psi (7kgf/cm²) and 250psi (18kgf/cm²).

A third aspect of the present invention is the integration of both processes to generate a process for the alkylation and transalkylation of aromatic and/or polyaromatic compounds for the selective monoalkylation of alkylaromatic compounds comprising at least one reactive catalytic distillation column comprising:
at least one reaction zone I containing at least one catalytic bed comprising a catalyst comprising zeolite with SAR of at least 60:1;
at least one reaction zone II containing at least one catalytic bed comprising a catalyst comprising zeolite with SAR of at least 80:1;
wherein the zone II precedes the zone I according to the main feeding stream of the aromatic compound;
wherein said process comprises:
   adding at least one feeding stream of aromatic compound in the zone II in upstream;
   adding at least one feeding stream of olefin in the zone I in downstream.

In an embodiment, it is a process for the alkylation and transalkylation of aromatic and/or polyalkylaromatic compounds for the selective monoalkylation of alkylaromatic compounds comprising at least one reactive catalytic distillation column comprising:
at least one reaction zone I (Alkylation) containing at least one catalytic bed comprising a catalyst comprising dealuminized modified zeolite-USHY with SAR of at least 60:1, in an embodiment, the SAR is higher than 70:1 and until 80:1, doped with 5 - 10% niobium and tin;
wherein the aromatic compound permeates the catalytic bed upstream and the olefin permeates in the catalytic bed downstream in the reactive distillation column;
at least one reaction zone II (transalkylation) containing at least one bed comprising a catalyst comprising dealuminized modified zeolite-USHY with SAR of at least 80:1, in an embodiment, the SAR is at least 80:1 and until 100:1, doped with 5 - 10% niobium and tin.
wherein the aromatic compound permeates into the catalytic bed upstream and the polyalkylaromatic downstream in the reactive distillation column.
wherein the II zone precedes the I zone according to the main feeding stream of the aromatic compound;
wherein said process comprises:
   adding at least one feeding stream of an aromatic compound in the zone II upstream;
   adding at least one feeding stream of alpha or internal monounsaturated olefin, linear or branched containing from 1 to 12 carbon atoms in the zone I downstream.

In an embodiment the production is continuous.

In an embodiment said process further comprises side reactors of reaction preset adjacent to reactive catalytic distillation column.

In an embodiment the side reactors of reaction preset comprise ratio of benzene to olefin ranging from 5:1 to 30:1, space velocity (LHSV) ranging from 6 to 9 h⁻¹.

In an embodiment the catalytic beds, installed inside the reactive distillation column, present molar ratio of aromatic compound to olefin ranging from 20 to 120, and space velocity LHSV ranging from 6 to 15h⁻¹.

In an embodiment of the present invention is to provide a process of the above mentioned type and to allow, in an efficient and economical way, continuously and permanently regenerating and recovering the catalytic activity, increasing the catalyst life cycle.

In a preferred embodiment said process further comprises at least one fractionating and purificating zone of the stream of the monoalkylaromatic compound resulting from the process.

In a preferred embodiment the monoalkylaromatic compound is of formula (I) 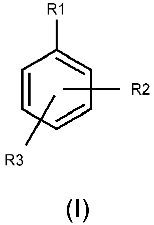 wherein when R1 is H and R2 is H and R3 is a linear or branched alkyl group containing from 1 to 12 carbon atoms.

In an embodiment the aromatic compound feeding stream is comprised of aromatic compound of formula (II) 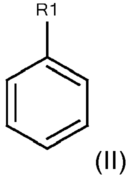 wherein R1 is H, CH₃, C₂H₅ or C₃H₇;
wherein the flow rate of the aromatic compound feeding stream is in the range from 20 to 40 m³/h.

In an embodiment the olefin feeding stream is made up of alpha or internal monounsaturated olefin, linear or branched containing from 1 to 12 carbon atoms, and the flow rate of the olefin feeding stream is in the range of 1 to 3 m³/h.

In an embodiment said process further comprises multipoint feed of reactive catalytic distillation column to the inlet of the feeding streams.

In an embodiment the zeolite is a zeolite USHY with SAR 60:1 - 80: 1 and a dealuminated and modified zeolite USHY SAR 80:1 - 100: 1, doped with 5 - 10% niobium and tin.

In an embodiment, the zeolite is a zeolite USHY with SAR higher than 70:1 and until 80:1 and a modified and dealuminated zeolite USHY with SAR between 80:1 - 100:1 doped with 5 - 10% niobium and tin.

The process in question can be performed continuously with the use of more than one reactive distillation column, and also compresses with high productivity in a limited production scale variable to 20.000t/year and less than the capacity of industrial units operating in the global market in the production of alkylbenzenes "commodities" for the surfactants segment among others. The plant required to achieve the process of the invention may be provided with automation and control system, industrial and environmental safety, low waste and effluents, with material costs and streamlined energy and competitive constructive investment and multi-purpose in production of various types of alkylaromatics using alpha olefins, and, in an embodiment, petrochemical benzene such as the main raw materials and selective solid acid catalysts in the production of a variety of alkylbenzenes with specifications and physicochemical characteristics most suitable for the market lubricating oil of cooling systems with hermetic compressors with high efficiency.

### Brief Description of the Figures

In order to better define and clarify the content of the present application, the following figures are presented:
Figure 1 represents a profile of the conversion rate versus time in the alkylation reaction of benzene with 1-octene.
Figure 2 represents a profile of the conversion rate versus time in the alkylation reaction of benzene with 1-decene.
Figure 3 represents a profile of the conversion rate versus time in the alkylation reaction of benzene with 1-dodecene.
Figure 4 represents a GC / MS analysis of conversion rate profile vs. time in the alkylation reaction of benzene with 1-decene and 1-dodecene.
Figure 5 represents a GC / MS analysis of the product of alkylation of benzene with 1-decene.
Figure 6 represents a GC/MS analysis of the product of alkylation of benzene with 1-dodecene.
Figure 7 represents a portion of the reactive distillation column of the present invention having multiple catalytic beds and multiple side reactors of preset.
Figure 8 represents one configuration of reactive distillation column of the present invention operating in conjunction with the fractionating and purificating zone of the stream of the resulting monoalkylaromatic compound.
Figure 9 represents one configuration of the integrated reactive distillation column with a fractionating distillation system comprising a distillation column.
Figure 10 represents the fractionating distillation system which allows the overhead, bottom and side outlets stream separation, so as to lead to a better energy use in the process.
Figure 11 represents a comparative viscosity profile of oils comprising compounds that may be produced by the process of the present invention in comparison to an oil used in the art (DetenLAB 240).

### Detailed Description of the Invention

In the context of the present invention, the "reactive catalytic distillation column" should be understood as a distillation column of the aromatic compound permanently upstream containing internally catalytic beds and side reactors with the same catalysts, separated by alkylation reaction zone (I) and transalkylation reaction zone (II) and by feeding downstream olefin compounds in the reaction zone (I) and by feeding downstream polyalkylaromatic compounds in the reaction zone (II), operating in a variable condition of pressure, flow rates, temperatures and suitable recycling rates for each production scale and type of desired alkylaromatic.

In the context of the present invention, the "catalytic bed" should be understood as an appropriate set of supporting and fixing the solid catalysts within the reactors or reactive catalytic distillation columns, allowing the diffusion of the reagents, the adsorption and desorption of products occurring from pores to surface and from surface to pores, favoring the reaction and its chemical phenomena in the active sites present in the inner and outer surface of the catalysts.

In the present context, the term "mesoporous" should be understood as the crystalline structure of catalysts having an average pore diameter > 20 Å.

In the context of the present invention, the term "reaction zone" should be understood as a set of fixed bed containing catalysts of the same type in order to promote a given chemical reaction of interest.

In the context of the present invention, the "space velocity" (LHSV) should be understood as the quotient of the volumetric flow rate of reactants divided by the reactor volume (or the volume of the catalytic bed) indicates how many volumes of feedstock in the reactor by a unit of time (one reactor with a space velocity of 7h⁻¹ is capable of processing load equivalent to seven times the reactor volume per hour. It is commonly regarded as the reciprocal of residence time in the reactor.

In the context of the present invention, the "preset side reactors" should be understood as reactors containing a catalytic fixed bed of the same type of that the reaction zone of the reactive catalytic distillation column that is installed, being the feeding port and promoting a premixing of reagents and giving a start in the desired reaction in a very well controlled condition before the direct feeding in reactive catalytic distillation column

### Selection of Modified Acid Solid Catalysts

The study in the present invention initially focused on the selection of acid solid catalysts, commercially available in the global market, to present an excellent catalytic activity for the alkylation reaction of benzene with linear olefins C₆-C₁₆, such as for example C₆-C₁₂, having a high conversion, selectivity and yield in the production of corresponding monoalkylbenzenes.

The alkylation reaction of benzene with C8 alpha olefins (> 95% m) C10 (> 95% w) and C12 (> 95% w) was adopted as a reference for the test of catalytic activity in the selection of acid solid catalysts for this reaction in the reaction conditions: benzene/olefin molar ratio of 10:1; catalyst 10% m (relative to the olefin); temperature of 140°C for Olefin C8 and C10; temperature of 180°C for Olefin C12, pressure <10 kg/cm²; time of 120 minutes, using an Autoclave Reactor Parr Instruments reactor.

More than 15 kinds of commercial catalysts were tested in the above-mentioned conditions with a great diversity in their physicochemical characteristics concerning the crystal structure, type of molecular sieves, pore size, SiO₂/Al₂O₃ molar ratio, nominal cations (H⁺, NH₄⁺; Na⁺), sodium content and surface area of 100-900 m²/g. The catalysts used in this selection were classified by pore size of its structure and two supported metal catalysts TiO₂, Nb₂O₅ were also used and were classified as shown in Table I.

**Table I**

| **Types of Tested Catalysts** | |
|---|---|
| **Pore Size** | **Catalysts** |
| Small (< 4A) | Zeolites A |
| Medium (4-6A) | TS-1; ZSM-5; IPO-11; ZSM-12 |
| Large (6-8A) | AIPO-5; Zeolites X, HY; Beta Zeolite (H-BEA 25); CP-814 E; CP-814 C |
| Super large (>8A) | AlPO-B;VPI-5 |
| Mesopore (>20A) | MCM-41 |
| Supported metalic | TiO2 Nb2O5 |

Table II lists the main catalysts selected after results of "spot tests" of the catalytic activity. The other catalysts showed no activities for alkylation reaction of benzene or showed low activities with unsatisfactory conversions.

**Table II - Conversion vs. Catalysts**

| Catalyst | Temperature | Conversion | Selectivity | Yield |
|---|---|---|---|---|
| Without Catalyst | 120°C | | | |
| TiO₂ | 120°C | 8% | | |
| | 150°C | 4% | | |
| ZSM-5 | 120°C | | | |
| | 150°C | | | |
| | 170°C | 25% | | |
| | 190°C | 83% | | |
| Iron supported on coal | 150°C | 8% | 100% | 8% |
| | 170°C | 16% | 54% | 9% |
| | 190°C | 42% | | |
| Niobium supported on alumina | 150°C | | | |
| | 170°C | 13% | | |
| | 190°C | 17% | | |
| Niobium supported on coal | 150°C | 8% | | |
| | 170°C | 17% | | |
| | 190°C | 29% | | |
| Hydrotalcite | 150°C | 19% | | |
| | 170°C | 15% | | |
| | 190°C | 28% | | |
| Changed Modern ite | 150°C | 9% | | |
| | 170°C | 16% | | |
| | 190°C | 23% | | |
| Zeolite HY SAR=6 | 120°C | 6% | | |
| | 150°C | 13% | | |
| Beta Zeolite | 150°C | 46% | 97% | 44% |
| | 170°C | 73% | 96% | 70% |
| | 190°C | 91% | 98% | 89% |

Beta zeolites H-BEA 25 showed no conversion, the CP-814E conversion <9%, Beta CP-814 conversion from 91% to 190°C, however their deactivation was very fast, also demanding temperature above 190°C. The studies were concentrated on zeolites HY SAR 6 catalysts for presenting reaction activity at low temperature 120-150°C and allow greater flexibility in adjustments and changes in its structure and physicochemical properties, the dealumination techniques and doping with metal cations.

It was observed a large initial activity of these catalysts "zeolites HY SAR 6:1, causing an accelerated decline in conversion and selectivity, and finally ending with its deactivation. This was justified on the basis of secondary reactions shifting the conversion to form polyalkybenzenes type (C8- Bz-C8, C10-Bz-C10 and C12-Bz-C12) and polymers result of dimerization of olefins among others.

It was evident that these byproducts caused blocking and clogging the pores of the catalyst drastically reducing its reactive activity, compromising the conversion and selectivity for monoalkylbenzene. It was observed the formation of elementary coke (carbonization) due to the mild reaction conditions with temperatures below 200°C. Adjusting the benzene/olefins ratio to 20:1 minimized the secondary reactions, but it did not prevent its deactivation.

The catalysts Zeolites - HY SAR <10: 1 partially meet this alkylation reaction for the physicochemical size and characteristics of the tested and interest reagents molecules. There is great commercial availability of this type of catalyst, however, they do not perform satisfactorily.

It was necessary to promote studies on modification of the crystalline structure of these Zeolites by hydrothermal dealumination technique having with dealuminating agent, alkalized water vapor with ammonia (NH₃), reducing the content of Aluminum (Al) in the zeolite-HY structure for different values of SAR Si/Al (5:1 to 100:1) seeking to identify the optimal (SAR) for reaction with a more appropriate acid strength to the size of the molecules of the reagents (benzene + alpha olefins C8; C10 and C12 pure), evaluating the impact of this process in the catalytic activity of each modified species in relation to the starting zeolite.

The process of modification of the molecular sieves by dealumination basically consists of removing and reducing part of aluminum atoms from the crystalline structure in a controlled condition, preserving its main reaction characteristics, thus promoting changes in the catalyst directed to the conversions and selectivities according to the size and physicochemical characteristics of the molecules and the reaction conditions used.

The zeolite-HY (Commercial) for selected starting had a Si/Al (SAR) molar ratio <5; Nominal cations H+; Sodium (Na₂O) <3,0% m; Average pore diameter <6-8Å; Surface area of 700-800m²/g, being dealuminated for several rations of Si/Al: (5-10), (10-20), (20-30), (30-40), (40-50), (50-60), (60-70), (70-80), (80-100) and (> 100) using the hydrothermal procedure of dealumination by removal of aluminum atoms to the structural modification of the zeolites-HY.

### Examples of embodiments of the process

Samples of the catalysts (Zeolites-HY 6-8Å) were previously dehydrated in a vacuum oven at 300°C for 6 hours. During this step, a slow dealumination has started using the water of hydration of the zeolite, so that it can produce its hydrolysis. The dealumination was carried out in the presence of water vapor and ammonia (NH₃) at low concentration (1% v/v) between 2 and 6 hours, at a temperature between 550 and 600°C. After this time the addition of ammonia gas was cut and water vapor only maintained for an additional 2 hours at the same temperature to remove the ammonia residue from the catalyst. Subsequently, the water vapor stream was also cut and the samples were cooled to 150 ° C under a nitrogen flow, drying the samples for 3 hours, after which they were calcined in an oven at a temperature of 650°C for 5 hours.

The duration in these conditions has determined a greater starting zeolite dealumination being prepared for various samples to obtain zeolite with SAR ranging from 5:1 to > 100:1.

It was not used the technique of dealumination by aluminum exchange by a silicon atom from an external agent such as SiCl₄ and (NH₄) ₂SiF₆, to avoid the presence of species of fluorinated and chlorinated compounds as trace contaminants in the zeolite structure, which would be undesirable.

It was obtained polydisperse zeolites with well distributed micropores and macropores, with low acidity and more optimized while maintaining the crystallinity with higher tolerance to moisture and more thermally stable, having a much more stable catalytic activity with a much slower deactivation, increasing life of these catalysts. The zeolite HY modified by this technique shows a more appropriate activity for the mild reaction conditions for the size (C8 to C12) and nature of the molecules involved in these reactions with greater stability of the catalysts.

Dealuminated samples were subjected to the reference experiments in the alkylation of simple aromatic compound, for example, benzene, with linear olefins C8, C10, C12 in the evaluation of conversion and selectivity in the production of the corresponding monoalkylated by checking the catalyst activity summarized in Table III.

**Table III: Conversion vs. SAR (Zeolite HY SAR 6:1 Dealuminated)**

| **Catalyst** | **Temperature** | **Conversion** | **Selectivity** | **Yield** |
|---|---|---|---|---|
| Without Catalyst | 120°C | - | | |
| Zeolite HY SAR=6 | 120°C | 6 | | |
| | 150°C | 13% | | |
| Zeolite HY SAR=14 | 120°C | | | |
| | 150°C | | | |
| | 170°C | 27% | | |
| | 190°C | 55% | | |
| Zeolite HY SAR=29 | 150°C | 29% | | |
| | 170°C | 40% | | |
| | 190°C | 62% | | |
| Zeolite **HY SAR=80** | **150°C** | **63%** | **98%** | **61%** |
| | **170°C** | **88%** | **96%** | **84%** |
| | **190°C** | **98%** | **96%** | **94%** |
| Zeolite **HY SAR=100** | **150°C** | **58%** | **92%** | **53%** |
| | **170°C** | **78%** | **93%** | **73%** |
| | **190°C** | **90%** | **94%** | **85%** |
| Zeolite HY SAR>10 | 150°C | | | |
| | 170°C | 19% | | |
| | 190°C | 33% | | |

The results demonstrated that more selective and stable dealuminated zeolite HY catalyst for the alkylation reaction was the one having a SAR molar ratio of (60:1 to 80:1), with a crystalline structure with polydisperse pores (large, medium to micropores) as well as the catalyst with SAR between (80:1 - 100:1) that showed most suitable for both the alkylation reaction and particularly for the transalkylation reaction.

Superactivity and accelerated deactivation of the catalyst were partially solved by reducing the initial catalytic activity for the reduction of acidity by increasing the Si/Al ratio from 5:1 to the range of (60:1 to 80:1) and (80:1 - 100:1) and by diffusion solution of the molecules of reactants, products and generated byproducts delaying the clogging of the pores of the catalytic beds, thus prolonging the activity of the catalyst. The selected catalysts modified by the quoted dealumination process presented both appropriate reduction of its initial activity as well as a favorable and desired polydisperse structure for the studied reaction.

Dealuminated zeolites HY with high SiO₂/AlO₃ (SAR) molar ratio exhibited a more controlled and adequate catalytic activity for the alkylation reactions of benzene with lighter olefin molecules such as C₆ to C₁₂, both as reactants and monoalkylaromatic and polyalkylaromatic products diffuse more easily into the open matrix structure than in the microporosity of the zeolites (<4 A). Under these circumstances, the zeolite which undergoes a dealumination (loss of aluminum atoms of structure) has an adequate reduction of the catalytic activity by reducing its initial acidity, reducing its deactivation by pore blockage by polyalkylaromatic molecules formed in this reaction, also reducing coke formation, thus improving the conversion and selectivity in monoalkylaromatics.

Looking to give greater reaction stability e to increase the life of catalysts selected after dealumination process by reducing its deactivation by the formation of polyalkylaromatics, these zeolites were modified by the addition of 5 to 10% of niobium and tin, that is, doped with these metals to promote a reaction with a higher conversion and selectivity for greater reaction time, increasing the life of the catalyst, delaying its deactivation, thereby increasing the selectivity for monoalkylated, with a significant improvement in the evaluation reaction of this catalytic activity (Table IV).

**Table IV: Modified and Dealuminated Zeolite USHY + 5% - 10% Nb-Sn. (Alkylation)**

| Catalyst | Temperature | Conversion | Selectivity | Yield |
|---|---|---|---|---|
| Zeolite USHY SAR 60 + (5%-10%) Nb - Sn. | 150°C | 85% | 95% | 81% |
| | 170°C | 90% | 94% | 85% |
| | 190°C | 98% | 94% | 92% |
| Zeolite USHY SAR 80 + (5%-10%) Nb - Sn. | 150°C | 90% | 98% | 88% |
| | 170°C | 97% | 96% | 93% |
| | 190°C | 99% | 96% | 95% |
| Zeolite USHY SAR 100 + (5%-10%) Nb - Sn. | 150°C | 85% | 93% | 79% |
| | 170°C | 91% | 94% | 86% |
| | 190°C | 98% | 93% | 91% |

It was observed a significant improvement in the life of the catalyst and on its deactivation in relation to the starting catalyst used after its structural modification by dealumination, which improvement was justified by the appropriate reduction of the initial catalytic activity, which also promoted, very quickly, the secondary reactions and premature clogging of the pores of the catalytic beds.

Modified and selective acid solid catalysts for:

### Alkylation Reaction

- Example of catalyst: Modified "Dealuminated" Zeolites USHY, SAR (Si/Al - 60:1 to 80:1), 5 - 10% niobium and tin, Sodium Content <0,05%, Total Acidity <100 (µmol, pyridine/g); surface area 700-800m²/g,

### Transalkylation Reaction

- Example of catalyst: Modified "Dealuminated" Zeolites USHY, SAR (Si/Al - 80:1 to 100:1), 5 - 10% niobium and tin, Sodium Content <0,05%, Total Acidity <100 (µmol, pyridine/g); surface area 700-800m²/g.

Table V shows the results of the alkylation reaction of benzene with C₁₂ olefin under the conditions tested with the mixture of zeolites SAR (60-80) and SAR (80-100) doped with 5 to 10% of niobium and tin, as well as simultaneous transalkylation reaction with Zeolites SAR (80-100) doped with 5 to 10% of niobium and tin, with an increase of conversion and yields in general.

**Table V: Modified and Dealuminated USHY Zeolite + 5% - 10% Nb - Sn.**

| **Alkylation** | | | | |
|---|---|---|---|---|
| **Catalyst** | **Temperature** | **Conversion** | **Selectivity** | **Yield** |
| Zeolite USHY SAR 60-80 + (5%-10%) Nb - Sn. | 150°C | 87% | 97% | 84% |
| | 170°C | 95% | 95% | 90% |
| | 190°C | 99% | 96% | 95% |
| Zeolite USHY SAR 80-100 + (5%-10%) Nb - Sn. | 150°C | 87% | 96% | 84% |
| | 170°C | 94% | 95% | 89% |
| | 190°C | 98% | 94% | 92% |

| **Transalkylation** | | | | |
|---|---|---|---|---|
| Zeolite USHY SAR 80-100 + (5%-10%) Nb - Sn. | 150°C | 93% | 98% | 91% |
| | 170°C | 97% | 97% | 94% |
| | 190°C | 99% | 97% | 96% |

The alkylation reaction of benzene with alpha olefins C8 (> 95% m), C10 (> 95% m) and C12 (> 95% m) was adopted as a reference for the test of catalytic activity in the selection of acid solid catalysts for this reaction in the reaction conditions: benzene / olefin molar ratio of 10:1; catalyst 10% m/m (with respect to the olefin); temperature of 140°C for Olefin C8 and C10; temperature of 180°C for Olefin C12, pressure <10 kg / cm2; time of 120 minutes, using an autoclave reactor-Parr Instruments reactor.

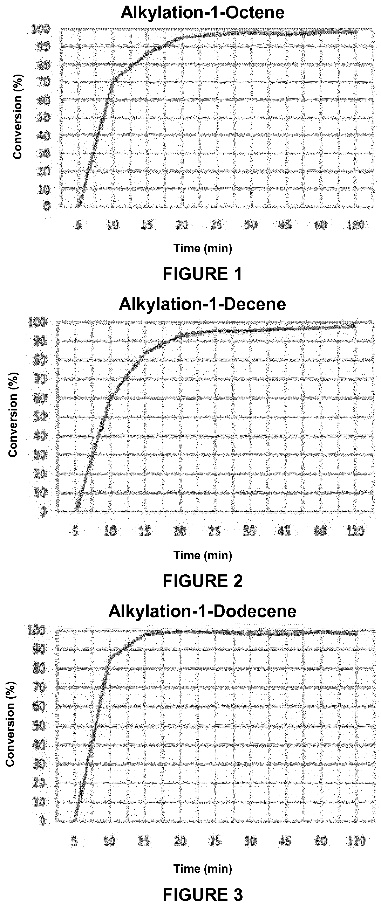

Figures 1, 2 and 3 illustrate the conversion, selectivity and yield rates in different reaction times for alkylation with octene (C8), decene (C10) and dodecene (C12) on these catalysts.

The conversion rate measures the transformation of all olefin mainly of mono- and polyalkylated alkylaromatics and in a lesser extent in the olefinic polymers. The selectivity rate measures the formed percentage of the product of interest relative to the total converted, in this case the product of interest would be the alkylaromatic products of monoalkylation. The yield rate measures how much of the product of interest was formed in the original quantity of reagents. Graphics showing the profile conversion with 1-octene, 1-decene and 1-dodecene can be found respectively in Figures 1, 2 and 3. Tables VI, VII and VIII below illustrate the conversion, selectivity and yield rates as a function of time in the alkylation with 1-octene, 1-decene and 1-dodecene, respectively:

**Table VI: Conversion, selectivity and yield rates as a function of time in alkylation with 1-octene as can be seen in figure 1.**

| Time (min) | Conversion (%) | Selectivity (%) | Yield (%) |
|---|---|---|---|
| 5 | 0 | 0 | 0 |
| 10 | 70 | 96 | 55 |
| 15 | 86 | 94 | 80 |
| 20 | 95 | 94 | 84 |
| 25 | 97 | 94 | 87 |
| 30 | 98 | 96 | 89 |
| 45 | 97 | 94 | 90 |
| 60 | 98 | 95 | 93 |
| 120 | 98 | 93 | 88 |

**TableVII: Conversion, selectivity and yield rates as a function of time in alkylation with 1-decene as can be seen in figure 2.**

| Time (min) | Conversion (%) | Selectivity (%) | Yield (%) |
|---|---|---|---|
| 5 | 0 | 0 | 0 |
| 10 | 60 | 96 | 57 |
| 15 | 84 | 94 | 79 |
| 20 | 93 | 94 | 85 |
| 25 | 95 | 94 | 86 |
| 30 | 95 | 93 | 88 |
| 45 | 96 | 91 | 87 |
| 60 | 97 | 91 | 89 |
| 120 | 98 | 94 | 88 |

**Table VIII: Conversion, selectivity and yield rates as a function of time in alkylation with 1-dodecene as can be seen in figure 3.**

| Time (min) | Conversion (%) | Selectivity (%) | Yield (%) |
|---|---|---|---|
| 5 | 0 | 0 | 0 |
| 10 | 85 | 94 | 80 |
| 15 | 98 | 93 | 91 |
| 20 | 100 | 95 | 95 |
| 25 | 99 | 94 | 98 |
| 30 | 98 | 99 | 98 |
| 45 | 98 | 94 | 93 |
| 60 | 99 | 94 | 92 |
| 120 | 98 | 93 | 92 |

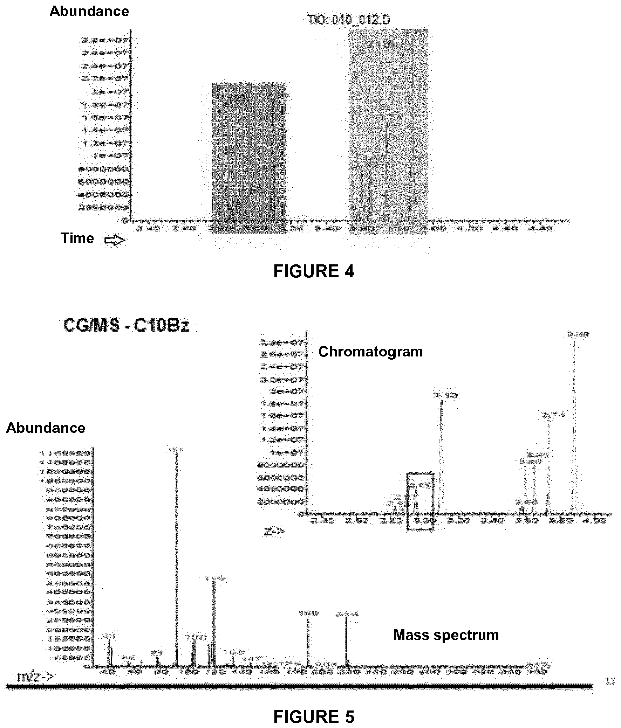

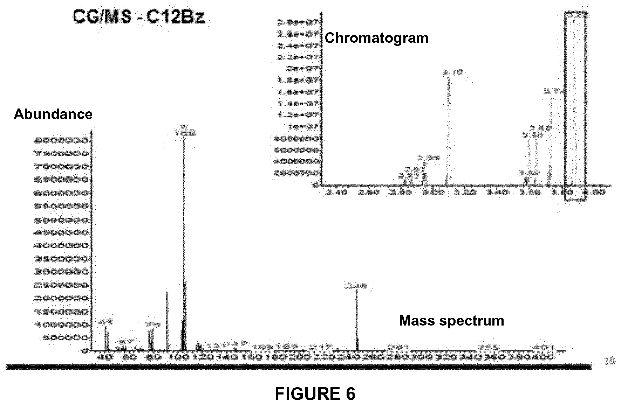

Figures 4, 5 and 6 show a profile of the characteristics of the product alkylbenzene C10 and C12 by gas chromatography attached to mass spectrometry.

As can be seen, the dealumination by hydrothermal process for controlled removal, for the modification of zeolites HY using the water vapor and ammonia gas as dealuminating agent, proved to be an effective method. The removal of aluminum could be confirmed by increasing the Si / Al by changing the SAR ratio (Si/Al) of 5:1 for values from 60:1 to 80:1 for the alkylation reaction and values from 80:1 to 100:1, for the transalkylation reaction, with a significant reduction in acidity without a significant change in the crystaline structure of the starting material.

The addition of niobium and tin metals gave greater stability of reaction, the addition of these metals gave a more balanced acidity by set distribution of Bronsted-Lewis acid sites, the acid strength suitable for studied alkylation and transalkylation reactions by improving the conversion and selectivity, delaying its deactivation and improving the life cycle of these catalysts.

### Integrated Process of Alkylation and Transalkylation in Reactive Distillation Columns

The process in question facing the alkylation reaction of simple aromatic compound which may be selected from benzene, toluene, ethyl benzene, cumene and mixtures thereof, is carried out in a reaction system, defining at least one ***reactive catalytic distillation column*** with ***multiple vertical catalytic beds*** and spaced fixed within the column and with ***multiple presets side reactors*** containing the appropriate catalyst for each reaction zone to be described later.

According to the invention, the process comprises the step of feeding in reactive distillation column a load of an alkylating agent, defined by at least one straight chain olefin, in the form of an alkyl group containing from 6 to 12 carbon atoms, for example, a straight chain alpha-olefin; a charge of simple aromatic compound defined above; and a load of a heterogeneous inorganic catalyst comprising zeolite USHY modified by dealumination process as previously described in this report SAR (60:1 - 80:1) doped with 5 to 10% of niobium and tin in the alkylation zone and with SAR (80:1 - 100:1) doped with 5 to 10% of niobium and tin in the transalkylation zone.

The catalyst is arranged in each catalytic bed in a proportion between 5-10% by weight relative to the load of olefin (t/day) and being provided with simple aromatic compound in molar ratio of 5:1 and 120:1 by relative to the load of olefin, said process taking place at a temperature of 100 to 200° C, at a pressure of 100psi (7kgf/cm²) to 250 psi (18kgf/cm²) and a reaction time of 10 to 50 minutes to produce alkylated aromatic compound.

As already described above, the heterogeneous inorganic catalyst disposed in each catalytic bed is defined by a zeolite. In an embodiment, the zeolite is a zeolite USHY having a surface area between 200 and 800 m²/g.

The alkylation reaction carried out within the column produces alkylated aromatic compounds containing from one to three alkyl groups, each alkyl group having usually from 6 to 12 carbon atoms. In an embodiment, there is a higher selectivity for the production of monoalkylaromatic compounds.

In a first aspect, the present invention defines a process for producing alkylaromatic synthetic lubricating oils by alkylation and transalkylation reaction in reactive distillation column in the formula (I), 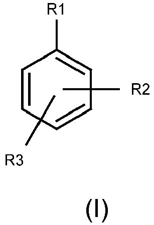 wherein:
- R1 is H, CH₃, C₂H₅ or C₃H₇;
- R2 and R3 are independently H or a linear or branched alkyl containing from 1 to 12 carbon atoms;
comprising the steps of:
- adding at least an alpha or inner monounsaturated olefin, linear or branched containing from 1 to 12 carbon atoms;
- adding an aromatic compound of formula (II) 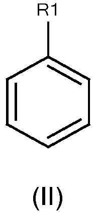 wherein R1 is H, CH₃, C₂H₅ or C₃H₇;
- over a calatyst comprising modified and dealuminated Zeolite USHY with SAR between 60:1 and 80:1.
- wherein the molar ratio of aromatic compound and olefin is between 5:1 and 120:1.

Yet another aspect of the present invention is the increase in the selective production of monoalkylaromatic compound by simultaneous transalkylation of polyalkylated compounds of formula (I) comprising the steps of:
- adding benzene;
- adding a polyalkylaromatic compound of formula (I) 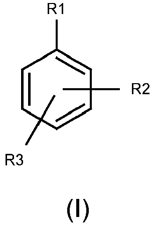 wherein R1, R2 and R3 are independently H or a linear or branched alkyl containing from 1 to 12 carbon atoms;
   wherein said polyalkylaromatic compound has at least two substitutions on the its aromatic ring;
- over a calatyst comprising modified and dealuminated zeolite HY with SAR between 80:1 and 100:1;
- wherein the molar ratio of benzene and the polyalkylaromatic compound is between 50:1 and 120:1.

It is yet another aspect of the present invention, an integrated process of alkylation and transalkylation of aromatic and/or polyalkylaromatic compounds for the selective production of monoalkylaromatic compounds comprising at least one catalytic reactive distillation column comprising:
- at least one alkylating reaction zone (I) containing at least one catalytic bed comprising a modified and dealuminated zeolite USHY catalyst with SAR between 60:1 and 80:1;
- at least one transalkylating reaction zone (I) containing at least one catalytic bed comprising a modified and dealuminated zeolite USHY catalyst with SAR between 80:1 and 100:1;
- wherein zone II precedes the zone I according to the main feeding stream of the aromatic compound;
- wherein said process comprises:
- adding in upstream zone II at least one feeding stream of an aromatic compound;
- adding at least one feeding stream of alpha or inner monounsaturated olefin, linear or branched containing from 1 to 12 carbon atoms in the downstream zone (I).

The alkylation and transalkylation process of aromatic compounds of the present invention comprises:
- at least of one reactive catalytic distillation column containing multiple presets side reactors, which is divided by reaction zones (I) and (II) each presenting respective fixed catalytic beds;
- this column is given the operational starting after being inventoried with the aromatic compound and set in an operational condition for permanently upstream distillation and being held in a condition of total reflux and recycle of the aromatic compound, at a temperature between 100 and 200°C and pressure <20kgf / cm². In this condition all the catalytic beds within the column will be in contact with the aromatic compound and a steam/liquid reaction condition suitable for the desired reaction.
- a feeding of at least one alkylating agent and part of recycle aromatic compound, the top product of the reactive catalytic distillation column, said feeding being made in the first preset lateral reactor containing a catalytic fixed bed of the same species of alkylation reaction zone, providing a pre-mixing of the reagents and giving a start in the desired reaction in a very well controlled reaction before the direct feeding in the reactive catalytic distillation column. The effluent of this side reactor feeds the column in the reaction zone (I) in the upper fixed bed and a second fixed bed arranged immediately below and spaced from the first one. This operation is repeated in the same way by the number of presets side reactors installed in the reactive column, with their effluent feeding the column from the corresponding catalytic beds and in the liquid phase of internal reflux in each bed;

- a feeding of a simple aromatic compound in the reactor, said feeding being made below the last lower fixed bed of reactive column, being maintained in permanent reflux and recycle;
- wherein each catalytic bed of the portion of reactive distillation column being provided from a load of an inorganic heterogeneous catalyst defined by a zeolite, such as a zeolite USHY, with SAR (60:1 - 80:1), preferably 5 -10% Nb - Sn and present in a proportion between 5 and 10% by weight, relative to the alkylating agent (t/day),
- wherein said column being internally maintained at a temperature of 100 to 200°C, at a pressure of 100psi (7kgf/cm²) to 250 psi (18kgf/cm²) for the production of the desired alkylated aromatic compounds.

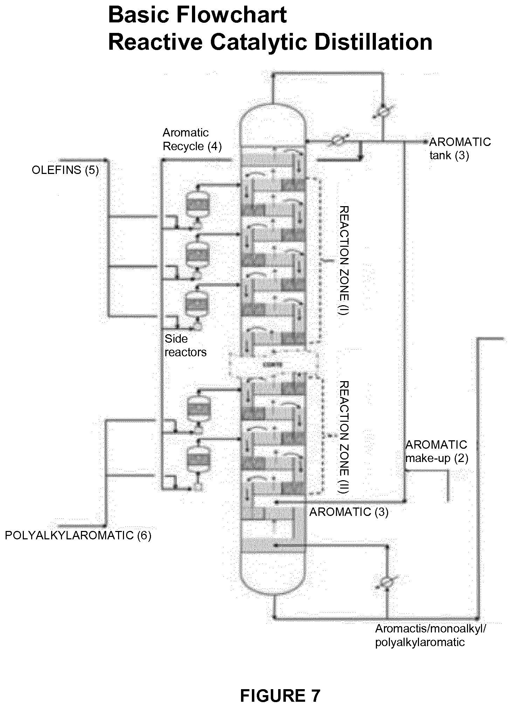

As can be seen in Figure 7, the portion of the reactive distillation column has multiple catalytic beds and multiple presets side reactors that may be, for example, in number of three or more, with a first catalytic bed provided in a portion top of the column, provided with at least one intermediate bed immediately below the first, and finally, a last alkylation catalytic bed. The catalytic beds are arranged spaced apart in a descending sequence to permeate upstream, the simple aromatic compound in molar excess, in the portion of reactive distillation column below the last alkylation catalytic bed, from a recycle aromatic line. At the same time, said alkylation catalytic beds permeate, dowstream, the alkylating agent from a controlled supply line, as the effluents of presets side reactors. The reactions conducted in the alkylation catalytic beds produce at least 90 % by weight of the monoalkylated alkylaromatic compound and the remainder of at least several isomers of polyalkylated alkylaromatic compounds.

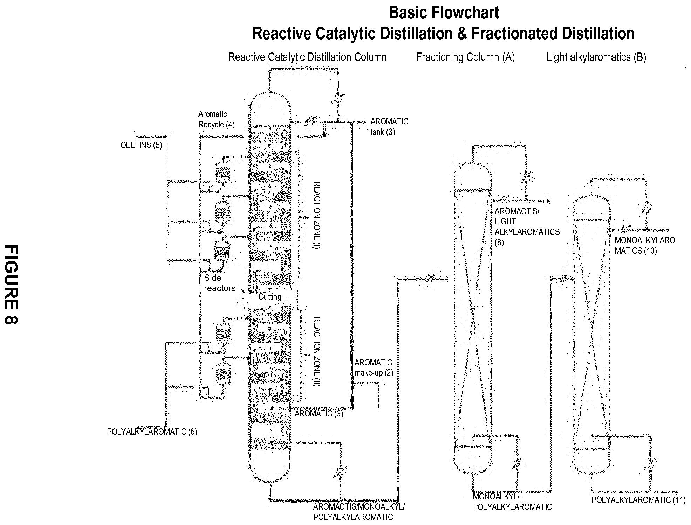

As shown in Figure 8, the monoalkylated compound and the polyalkylated compound produced in reaction zone (I) remain downstream in the column and to permeate in the catalytic beds of the reaction zone (II), which contains most suitable catalysts for the transalkylation reaction of polyalkylated, but also continues to make alkylation from possible unreacted alkylating agent in the previous step. This zone is no longer made the feeding of the alkylating agent but an adjustment in the feeding of the recycle aromatic compound in order to significantly increase the molar ratio of aromatic compound relative to the load of the alkylating agent of the above reaction zone to promote the transalkylation reaction of polyalkylated, that is, to reverse the monoalkylated into polyalkylated in this step, so it is possible to increase the selectivity of monoalkylated more than 95 % by weight, dramatically reducing the production of polyalkylated.

The operational adjustments to the reactive column aims to maintain a concentration of no greater than 10% of the aromatic compound in the bottom stream of the reactive distillation column, that together with the produced monoalkylated and no transalkylated polyalkylated are withdrawn from the bottom thereof for feeding and are separated in a fractionating distillation column (A) so that the aromatic compounds (max 10%) and the possible unreacted alkylating agents, as well as possible light alkylated formed in the reaction, are separated by the top of this column (A), and the bottom product is essentially composed of monoalkylated and polyalkylated.

The overhead stream of this column (A) is stored and recycled to the process, being and reprocessed preferably in the recycle stream of aromatic compound feeding the bottom of the reactive distillation column. The bottom stream of this column (A), comprising monoalkylated and polyalkylated feeds another fractionating column (B).

The fractionating column (B) will separate by the top stream the monoalkylated from the polyalkylated compounds, in the bottom stream of this column. The monoalkylated (Column B Top) are aligned and stored for specified product tanks which, after drying and additive with antioxidants, are ready for use as lubricating oils in cooling systems, and the polyalkylated (Column Fund - B) to process lung tanks to be or not again transalkilated in the reactive distillation column.

The polyalkylated aromatic compound is introduced into the reactive distillation column, by preset side reactors together with part of the recycle aromatic compound, above at least one transalkylation catalytic bed, from a polyalkylated line of process lung tank which stores the bottom product of the fractionating column (B).

The reactive catalytic distillation column carries, in its interior, in the transalkylation reaction zone (II) at least one fixed catalytic bed containing a load of zeolite catalyst, such as for example a zeolite USHY having a SAR ratio (80:1-100:1) preferably with 5 to 10% Nb - Sn permeating upstream, the simple aromatic compound - recycle stream - that is introduced into the reactive distillation column below the last transalkylation catalytic bed. At the same time, at least one transalkylation catalytic bed permeates downstream the polyalkylated compound to be transalkylted in monoalkylated aromatic compound, and the catalyst disposed in each catalytic bed of the reactive distillation column, in a proportion between 5-10 % by weight (t / day), compared to alkylating agent in the alkylation step in the reaction zone (I) and being provided the simple aromatic compound in the molar ratio of, for example, 40:1 to 60:1 relative to the load olefin cited above, feeding this transalkylation reaction zone (II). These olefins in this step (II) must be 100% reacted and converted into monoalkylated and polyalkylated in the alkylation reaction zone (I).

In an embodiment of the process, the system is kept under a temperature of 80 and 200°C, a pressure of 100psi (7kgf/cm²) to 250 psi (18kgf/cm²) and a reaction time of 10 to 50 minutes for the transalkylation of the polyalkylated aromatic compound.

In the embodiment illustrated in the basic flowchart in Figure 8, this reaction multi-purpose system can work in both in the form of simple alkylation without promoting the transalkylation, in the form of simultaneous alkylation and transalkylation, in the form of additional and simultaneous alkylation and transalkylation by reprocess of polyalkylated stored in the process lung tank, it is also possible to make operational adjustments driving the alkylation reaction for the production of polyalkylated, if of interest in the alkylation of other aromatic compounds with radicals CH₃, C₂H₅ and C₃H₇, as well as can also promote a increased production of polyalkylated by appropriate operational adjustments in the alkylation reaction zone (I), for example, by reducing the molar ratio of the reactants in order to drive the reaction to the production of polyalkylated of the same alkylating agent, and the simple aromatic compound used such as benzene and others.

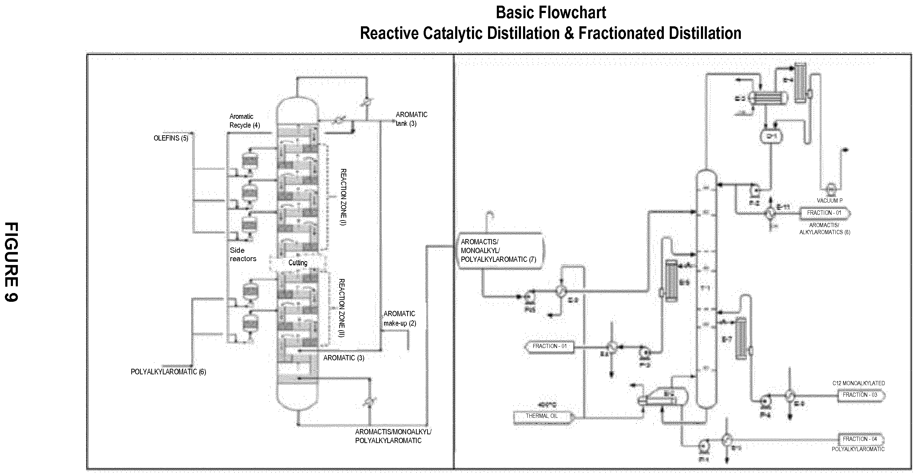

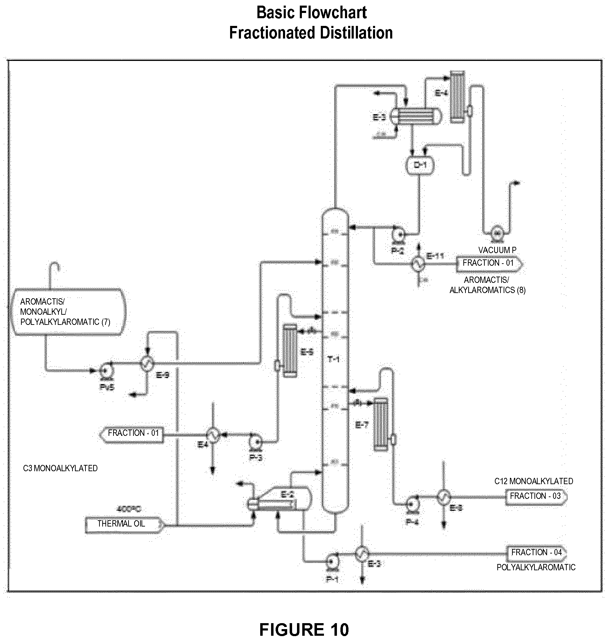

An ideal option would be an integrated reactive catalytic distillation system with a fractionating distillation system comprising one distillation column (Figure 9) designed to allow the separation of various chains: the top, bottom and side outlets enabling a better energy use in this process. (Figure 10)

The olefin employed is preferably a straight chain alpha-olefin having from 6 to 16 carbon atoms, preferably from 6 to 12 carbon atoms, more preferably from 6 to 10 carbon atoms, obtained by the process of ethylene oligomerization (Ziegler or SHOP), being processed separately by number of carbon C₆; C₈; C₁₀ and C₁₂ of high purity or mixtures thereof, adjusted in appropriate proportions. Other olefins may also be used, such as those obtained by the process of UOP Pacol™ - DeFine™, dehydrogenation of n-paraffins C₁₀ - C₁₃, separated and purified by the process, UOP Olex™ for the production of high purity internal linear olefins.

There may also be the option of direct alkylation of mixtures of olefins and paraffins, and other inert compounds directly feeding the reactive distillation column, and expanding the operational and reaction conditions, favoring the obtaining of higher conversions, selectivities and yields in the production of desired monoalkylaromatics. The presence of inert diluents fluid with boiling point above the aromatic compounds used and below the boiling point of the lighter monoalkylated, thus being also removed from the bottom of this column with the products of this reaction, it will directly contribute to drag, downstream between the catalytic beds, the alkylating agent and the products formed by acting directly in a larger area in the balance of this reaction. However, this option may result in a loss of production capacity of the reactive distillation column, as well as the fractionating column design (A) should be able to promote recycling in the movement of inert products, thus closing the system. These are options that should be evaluated as a further feature in a compact multi-purpose system as set forth.

In one embodiment of the process of the present invention, the reactive distillation column comprising at least 05 (five) preset side reactors contains at least 03 (three) alkylation catalytic beds in the reaction zone (I) and at least 02 (two) transalkylation catalytic beds in the reaction zone (II).

The configuration illustrated in Figure 8 comprises reactive catalytic distillation column of the recycle benzene in molar excess, containing 05 (five) presets side reactors, contains 03 (three) alkylation catalytic beds, reaction zone (I) and 02 (two) transalkylation catalytic beds, reaction zone (II) in the following sequence, from top to bottom:
- Zone (I) - 1°; 2° and 3° - alkylation catalytic bed: a zeolite, for example a modified and dealuminated zeolite USHY with SAR 60:1-80:1; doped with 5 to 10% of Nb - Sn.
- Zone (II) - 4° and 5° - transalkylation catalytic bed (with secondary alkylation): a zeolite, such as for example a modified and dealuminated zeolite USHY with SAR 80:1-100:1; with 5 to 10% of Nb - Sn.

It is recommended to install in parallel two reactive distillation columns, for operation on alternate cycles of reaction and regeneration of catalysts. However, the operational condition of the system with a single column has become quite as favorable in increasing the life of the catalysts according to their permanently operation in the presence of the aromatic compound in a large molar excess, still can cut the feeding of the alkylating agent in the alkylation zone and the polyalkylated in the transalkylation zone, leaving the whole system only with the aromatic compound in internal reflux and recycle, in a band operating condition for a period of 12 hours without any production of monoalkylated. In this condition the catalytic beds will be exposed in a very favorable reaction conditions for transalkylation process (in situ) of polyalkylated adsorbed on the catalyst, being converted into monoalkylated and permeating out of the crystalline structure of the catalyst, thus allowing a new operating production campaign of monoalkylated by simply feeding back the entire system.

The reactive distillation column is operated under appropriate and automated conditions in the control of temperatures, pressures, recycles rate and flow rates of the feedstock, to permit withdrawal from the top of the reactor of the aromatic compound, e.g., benzene in molar excess and which is recycled permanently to the reactive column closing one operating cycle, with the removal, by the fund, of the monoalkylated stream (e.g. monoalkybenzene) containing a polyalkylated residual (e.g. polyalkylbenzene) "non-transalkylated" more polyalkylated "produced" as by-product of the alkylation reaction, this stream also containing up to 10% by weight of the aromatic compound used which will feed into the fractionating distillation system at low pressure in the columns (A) and (B) after separation the overhead stream of column (A), and the bottoms stream from the column (B) of the monoalkylated, are continuously recycled to the reactive column, thus closing the operating cycle of both the alkylation reaction and the transalkylation reaction. The aromatic compound may also be selected from benzene, toluene, ethylbenzene and cumene, and a mixture thereof.

Also in the reactive distillation column, the alkylation and transalkylation catalytic beds are loaded with heterogeneous acid solid catalysts, modified and comprising a zeolite, such as for example a dealuminated zeolite-USHY with SAR (60:1-80:1) to the alkylation and with SAR (80:1-100:1) to the transalkylation.

Said catalysts are provided between 5 and 10% by weight in each catalytic bed in relation to the olefin load (t/day) and operating the column with a "LHSV" from 6 to 15h⁻¹, the aromatic compound being provided in the ratio of 5:1 to 120:1 also in relation to the olefin, preferably 20:1 to 40:1.

The process in question leads to the production of monoalkylated aromatic compounds at high conversions and selectivities, using mild reaction conditions: temperatures below 200°C, pressures below 250psi and reaction time below 60 minutes. The alkylation reaction with an olefin and a heterogeneous catalyst defined, for example, by a modified dealuminated zeolite USHY (SAR between 60:1-80:1) operating in continuous process and by campaign in meeting the demands of the desired lubricating oil type to be produced in a flexibility and production multi-purpose, that is, monoalkylbenzenes C₆; C₈, C₁₀, C₁₂, C₈/C₁₀, C₁₀/C₁₂ or polyalkylbenzenes derived from alkylation of toluene, ethylbenzene, cumene and its variants C₆; C₈, C₁₀, C₁₂, C₈/C₁₀ and C₁₀/C₁₂.

It is also possible to operate in a more favorable condition for the production of polyalkylated derived from alkylation of benzene and its variants C₆-BZ-C₆; C₈-BZ-C₈; C₁₀-BZ-C₁₀; C₁₂-BZ-C₁₂ and mixtures thereof, allowing to obtain the conversions above 99.5% and selectivity above 93% for monoalkylated products.

Under these conditions it is possible to produce mono- and dialkylated aromatic compounds, such as alkylbenzene, alkyltoluene, alkylethylbenzene, alkylcumene and a mixture thereof with high conversion and selectivity, allowing the development and the production of lubricating oils with several viscosities ranges most suitable for each type of high efficiency hermetic cooling compressor.

The alkylation of benzene with linear olefins by the used process not only leads to the formation of monoalkylated (about 90 to 95% yield), but also di- and trialkylated (i.e. also polyalkylated) (5 to 10% yield). In case of alkylation by heterogeneous process, the polyalkylated molecules may also be responsible for deactivation of the solid catalyst by blocking the pores of the crystalline structure, can also have the formation of coke, contributing to the deactivation and reducing the active surface area of the catalyst.

In the model adopted of simultaneously and continuous reactive distillation in the reaction and distillation, it was observed a significant increase in catalyst life as well as the conversion and selectivity in the alkylation and transalkylation reactions compared to other technologies.

The process of regeneration, in a first option, is recommended to stop the alkylation and transalkylation reaction, making the cut in the feeding of the "olefin" alkylating agent and polyalkylated, keeping the reactive distillation column in the same operating parameters under normal operation conditions of the whole set, distilling and recycling the aromatic compound, for example, benzene, at appropriate period ranging from 6 to 12 hours, thereby facilitating cleaning of the catalytic bed, recovering the active surface area of the catalyst, the polyalkylated "non transalkylated" which are blocking the pores of the catalyst crystalline structure, and consequently, hindering the access of reagents to the reaction active sites of the catalyst. This process already occurs typically during production. However, with the depletion of olefin reactant (alkylating agent) and only being held the feeding of the aromatic compound, for example, benzene, the transalkylation reaction of polyalkylated contained in the catalyst will be accelerated and more efficient for the production of monoalkylated. In an embodiment, the step for regenerating the catalyst wherein the feeding of the aromatic compound and the operational parameters on normal regime of the reactive distillation column are maintained and the feeding of the alkylating agent and the polyalkylated compounds are interrupted. In an embodiment, the step for regenerating lasts at least 6 hours.

For the regeneration of catalysts when the above process achieves conversion and selectivity rates at a level of economic involvement, depending on the variables consumption become high, the recharge of catalytic beds with new catalysts is desirable.

The discharged catalyst may also be subjected to process of regeneration by burning and removing the coke deposited thereon by controlled oxidation reaction with the mixture (N₂/O₂), by removing the coke in the form of CO₂. This is an operation that requires a dedicated system and much control to prevent sintering of the catalyst and its definite deactivation for the desired application.

The catalyst used in this work allows the transalkylation of bi and trialkylated, after its separation from the monoalkylated, is made by recycling the polyalkylated to the reactor, reducing the olefin charge in the same proportion of cited recycle charge and adjusting the aromatic/olefin molar ratio of 20:1 to 40:1, increasing the selectivity in the conversion to monoalkylated, if the desired production is of the monoalkylated. This feature is a big difference in the studied catalyst.

In the embodiment illustrated in Figures 7 and 8, the process is carried out in a compact system that can be operated in two ways: a) operation of the alkylation reaction without recycling of polyalkylated; b) operation of the alkylation reaction with recycling of polyalkylated to promote the "in situ" transalkylation reaction.

This process includes the polyalkylated compounds found in alkylated aromatic compound separated by fractional distillation and converted into monoalkylated compounds for their recycling in reactive catalytic distillation column in the transalkylation reaction zone (II) capable of promoting the both the alkylation and transalkylation reactions. The transalkylation reaction is achieved by the use of a selective acid solid catalyst, having thermal stability and Brönsted and/or Lewis acidity in the reaction conditions and provided in molar ratios of aromatic /(olefins + polyalkylated) in the range of 20:1 to 30:1, the transalkylation reaction being carried out at temperatures between 100°C and 200°C, at pressures of 7kgf/cm² to 18kgf/cm² for a time of 10 to 50 min.

There are several advantages over the prior art:
- 90% of the alkylation units are homogeneous catalysis by using HF and AlCl₃ catalysts, as detailed in the beginning of the text.
- 10% use heterogeneous catalysis Detal UOP with modernite doped with HF in reaction system with double alkylation reactor operating on/off per campaign alternating production and regeneration of the catalyst;
- It has large distillation columns to recycle 100% of excess of the unconverted n-paraffins compound to olefins by the PACOL process as well as the excess of the aromatic compound into the respective reactors, representing around 90% of the circulating inventory, with high energy consumption.
- Dedicated units for the production of alkylbenzenes for the surfactants segment without great operational flexibility in the production of other alkylaromatics;
- Large industrial facilities with high investments.
- High cost of maintenance, occupational and environmental safety.

The proposed process has many advantages in relation to both existing commercial processes.
- Heterogeneous catalysis integrating alkylation, transalkylation and regeneration of catalysts;
- Reactive distillation column, melting reactor and recycle column of aromatic compound;
- Greater industrial, occupational and environmental safety;
- Low waste generation and effluents;
- "Multipurpose" unit in the production of various types of alkylaromatic;
- Flexible operation in the adjustment of endless molar ratios of aromatic compound/Olefins;
- Simultaneous alkylation and transalkylation process;
- Process of regeneration not required with the loss of production or equipment duplicity;
- Compact unit with high productivity, with varying scale of production;
- Small competitive constructive investment.

The alkylaromatic lubricating oil obtainable by the process of the present invention has no restriction on the molecular structure of the alkylaromatic component. However, given the interest in increasing the long-term reliability of a cooling system, it is preferable to select an alkylaromatic containing from 1 to 3 alkyl groups, each alkyl group containing from 6 to 12 carbon atoms and the total amount of carbon atoms in the alkyl group being from 6 to 24.

Examples of alkyl group containing from 6 to 12 carbon atoms are hexyl (including all isomers), heptyl (including all isomers), octyl (including all isomers), nonyl (including all isomers), decyl (including all isomers), undecyl (including all isomers), dodecyl (including all isomers).

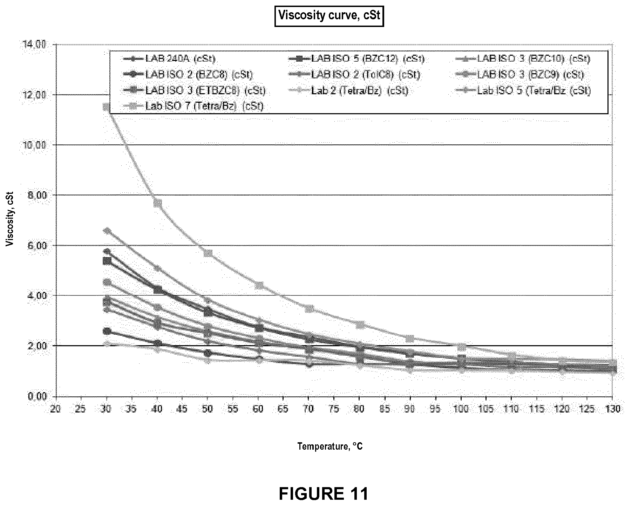

Considering the use of the lubricating oil in question together with refrigerants fluids containing hydrocarbon (HC), such as HC-600a and/or HC-290, it is desirable that the lubricating oil presents a viscosity in the range of about 2.0 to about 7.0 cSt at temperature of 40°C as observed in the viscosity curve of Figure 11 and table IX showing physicochemical characteristics of some examples of tested lubricating oils.

**Table IX - Physicochemical Characterization of the compounds analyzed in figure 11**

| **Physicochemical properties** | **Specification DetenLAB 240** | **Historical Value DetenLAB 240*** | **C12Bz (ISO 5)** | **C10Bz (ISO 3)** | **C8EtBz (ISO 3)** | **rC9Bz (ISO 3)** | **C8Bz (ISO 2)** | **CBTol (ISO 2)** |
|---|---|---|---|---|---|---|---|---|
| Color | Max. 1,0 | < 0,5 | < 0,5 | <0,5 | <0,5 | <0,5 | <0,5 | <0,5 |
| Density (g/cm3) | 0,845-0,870 | 0,860 | 0,852 | 0,860 | 0,867 | 0,866 | 0,860 | 0,860 |
| Viscosity 40°C (cSt) | 4,10-4,80 | 4,21 | 4,42 | 3,13 | 2,93 | 3,55 | 1,79 | 2,34 |
| Viscosity 100°C (cSt) | 1,25-1,60 | 1,33 | 1,40 | 1,34 | 1,31 | 1,26 | 0,91 | 1,03 |
| Miscibility HC-600a (°C) | Max. -50 | -70 | -70 | -70 | -60 | -60 | -60 | -60 |
| Flash point (°C) | Min. 135 | 142 | 144 | 126 | 120 | **114** | 106 | 120 |
| Combustion Point (°C) | Min. 145 | 150 | 152 | 134 | 128 | **118** | 110 | 121 |
| Acidity (mgKOH/g) | Max. 100 | < 0,01 | 0,02 | 0,01 | 0,03 | 0,02 | 0,02 | 0,02 |

In view of improving the property that prevents the generation of collapse of a hermetic compressor during a long period of operation, the alkylaromatic is preferably selected from those alkylaromatic oils containing 80% by weight or more, such as for example 85% by weight or more, or yet 90% by weight or more of linear alkylbenzene having a molecular weight of 162-415 g/mol.

In view of the stability and viscosity of alkylaromatics, straight chain monoalkylamino groups are preferable, since the linearity of the chain leads to a better lubricity (with low viscosity) and the existence of an alkyl group has a positive influence on the chemical stability of an alkylaromatic oil.

The number of alkyl groups on the aromatic compound is defined from 1 to 3. However, in view of stability and availability of alkylaromatic, it is more preferable to select an alkylaromatic containing one or two alkyl groups, i.e., a monoalkylaromatic, a dialkylaromatic or a mixture thereof. In an embodiment, there is a higher selectivity for the production of monoalkylaromatics.

It is also possible to apply not only the alkylbenzenes defined above, which have the same molecular structure, but also those with different molecular structures as long as the conditions are met containing alkyl groups from 1 to 3, each group preferably containing from 6 to 12 carbon atoms and the total amount of carbon atoms in the alkyl group being from 6 to 24.

The integrated system of alkylation, transalkylation and regeneration can also be designed and to be able to operate with olefinic alkylating agents in C₂ to C₄ for the production of special mono- and polyalkylaromatics, for example, polyisopropylbenzene and polyisobutylbenzene and others.

The above description of the present invention has been presented for purposes of illustration and description. Furthermore, the description does not intend to limit the invention to the form disclosed herein. Consequently, variations and modifications consistent with the above teachings, and the skill or knowledge of the relevant art, are within the scope of the invention. The procedures described above intend to explain the modes known for practicing the invention and to enable skilled technician to use the invention in such, or other modes and with various modifications required by the particular applications or uses of the present invention. It is intended that the present invention includes all modifications and variations thereof, within the scope described in the report and in the appended claims.

## Claims

**1.** Process for producing alkylaromatic compound, **characterized in that** it comprises the steps of
- adding at least one olefin;
- adding at least one aromatic compound;
- over a catalyst comprising zeolite with SAR of at least 60:1.

**2.** Process according to claim 1 **characterized in that** the catalyst comprises zeolite with SAR higher than 70:1.

**3.** Process according to claim 1, **characterized in that** the alkylaromatic compound is of formula (I) 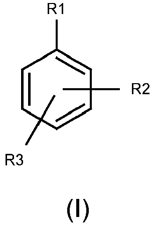 wherein:
- R1 is H, CH₃, C₂H₅ or C₃H₇;
- R2 and R3 are independently H or a linear or branched alkyl radical containing from 1 to 12 carbon atoms;
comprising the steps of:
- adding at least one alpha or inner monounsaturated olefin, linear or branched containing from 1 to 12 carbon atoms;
- adding at least one aromatic compound of formula (II) 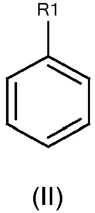 wherein R1 is H, CH₃, C₂H₅ or C₃H₇;
- over a calatyst comprising dealuminated modified zeolite HY with SAR higher than 70:1 and until 80:1; and
wherein the molar ratio of aromatic compound and olefin is between 5:1 and 120:1.

**3.** Process according to claim 2, **characterized in that** R1 is H and when R2 is H, R3 is a linear or branched alkyl group containing from 1 to 12 carbon atoms, and when R3 is H, R2 is a linear or branched alkyl group containing from 1 to 12 carbon atoms.

**4.** Process according to claim 1, **characterized in that** the total amount of carbon atoms linked to the aromatic ring of alkylaromatic compound is from 1 to 12.

**5.** Process according to claim 1, **characterized in that** the zeolite is a dealuminated modified zeolite-USHY.

**6.** Process according to claim 1, **characterized in that** the zeolite is doped with 5% - 10% of niobim and tin.

**7.** Process according to claim 1, **characterized in that** the temperature is between 80°C and 200°C and the pressure is maintained from 100 psi (7kgf/cm²) to 250psi (18kgf/cm²).

**8.** Process according to claim 1, **characterized in that** the catalyst presents surface area between 200 and 900m²/g.

**9.** Process according to claim 1, **characterized in that** the catalyst is in a proportion of 5-10% by weight relative to mass of olefin (t/day) arranged in at least one catalytic bed arranged in at least one reactive catalytic distillation column.

**10.** Process according to claim 1, **characterized in that** the aromatic compound permeates the upstream catalytic bed and the olefin permeates the downstream catalytic bed.

**11.** Process according to claim 2, **characterized in that** the alkylaromatic compound is of formula (I) 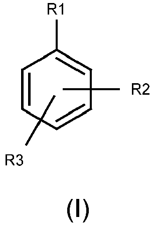 wherein
when R1 is H and R2 is H;
- R3 is a linear or branched alkyl containing from 1 to 12 carbon atoms;
or when R1 is CH₃, C₂H₅ or C₃H₇,
R2 is H; and
R3 is a linear or branched alkyl containing from 1 to 12 carbon atoms.

**12.** Process of transalkylation of polyalkylaromatics for selective monoalkylation of alkylaromatic compound, **characterized by** comprising the steps of:
- adding at least one aromatic compound;
wherein said aromatic compound has zero or one alkyl group linked to the aromatic compound;
- adding at least one polyalkylaromatic compound;
wherein said polyalkylaromatic compound has at least two alkyl groups linked to the polyalkylaromatic compound;
- over a catalyst comprising zeolite with SAR of at least 80:1.

**13.** Process according to claim 12, **characterized in that** the product of selective monoalkylation of alkylaromatic compound is of formula (I) 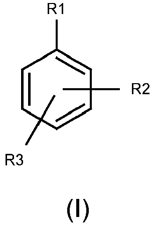 wherein when R1 is H and R2 is H and R3 is a linear or branched alkyl containing from 1 to 12 carbon atoms,
comprising the steps of:
- adding an aromatic compound;
wherein said aromatic compound has zero or one alkyl group linked to the aromatic compound;
- adding a polyalkylaromatic compound of formula (I) 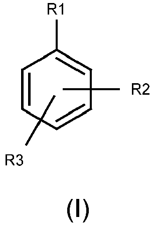 wherein R1, R2 and R3 are independently H or a linear or branched alkyl containing from 1 to 12 carbon atoms;
wherein said polyalkylaromatic compound has at least two alkyl groups linked to the polyalkylaromatic compound;
- over a catalyst comprising modified zeolite-HY with SAR of at least 80:1 and until 100:1,
wherein the molar ratio of aromatic compound to polyalkylaromatic compound is between 15:1 and 120:1.

**14.** Process according to claim 12, **characterized in that** the molar ratio of aromatic compound to polyalkylaromatic compound is between 20:1 and 40:1.

**15.** Process according to claim 12, **characterized in that** the zeolite is a dealuminated modified zeolite-USHY.

**16.** Process according to claim 12, **characterized in that** the zeolite is doped with 5% - 10% of niobium and tin.

**17.** Process according to claim 12, **characterized in that** the temperature is maintained between 80°C and 200°C and the pressure is maintained from 100psi (7kgf/cm²) to 250psi (18kgf/cm²).

**18.** Process according to claim 12, **characterized in that** the catalyst shows surface area between 200 and 900m²/g.

**19.** Process according to claim 12, **characterized in that** the aromatic compound permeates the catalytic bed upstream and the polyalkylaromatic compound downstream.

**20.** Process of alkylation and transalkylation of aromatic and/or polyaromatic compounds for the selective monoalkylation of alkylaromatic compounds **characterized by** comprising at least one reactive catalytic distillation column comprising:
at least one reaction zone I containing at least one catalytic bed comprising a catalyst comprising zeolite with SAR of at least 60:1;
at least one reaction zone II containing at least one catalytic bed comprising a catalyst comprising zeolite with SAR of at least 80:1;
wherein the zone II precedes the zone I according to the main feeding stream of the aromatic compound;
wherein said process comprises:
adding at least one feeding stream of an aromatic compound in the zone II upstream;
adding at least one feeding stream of olefin in the zone I downstream.

**21.** Process according to claim 20, **characterized in that** the reaction zone I contains at least one catalytic bed comprising a catalyst comprising zeolite with SAR higher than 70:1 and until 80:1 and the reaction zone II contains at least one catalytic bed comprising a catalyst comprising zeolite with SAR of at least 80:1 and until 100:1.

**22.** Process according to claim 20 **characterized in that** the product of selective monoalkylation is of formula (I) 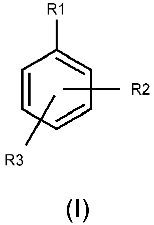 wherein when R1 is H and R2 is H and R3 is a linear or branched alkyl containing from 1 to 12 carbon atoms;
wherein the olefin feeding stream consists of olefin containing from 1 to 12 carbon atoms;
wherein the feeding stream of aromatic compound consists of aromatic compound of formula (II) 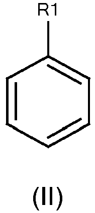 wherein R1 is H, CH₃, C₂H₅ or C₃H₇; and
wherein the feeding stream flow rate of the aromatic compound is in the range from 20 to 40 m³/h.

**23.** Process according to claim 20, **characterized by** comprising at least one step for regenerating the catalyst wherein the feeding of the aromatic compound and the operating parameters on normal regime of the reactive distillation column are maintained and the feeding of the alkylating agent and polyalkylated compounds are interrupted.

**24.** Process according to claim 23, **characterized in that** the step for regenerating lasts for at least 6 hours.

**25.** Process according to claim 20 **characterized in that** the reaction zone I comprises at least three catalytic beds and the reaction zone II comprises at least two catalytic beds.

**26.** Process according to claim 20 **characterized in that** the ratio of the aromatic compound feeding stream to olefin feeding stream is from 5:1 to 120:1.

**27.** Process according to claim 20 **characterized in that** the production is continuous.

**28.** Process according to claim 20 **characterized in that** it further comprises at least one fractionating and purificating zone of the stream of monoalkylaromatic compound resulting from the process.

**29.** Process according to claim 20 **characterized in that** it further comprises at least one reaction preset side reactor adjacent to the reactive catalytic distillation column.

**30.** Process according to claim 29 **characterized in that** it comprises at least five reaction preset side reactors.

**31.** Process according to claim 29, **characterized in that** it the reaction preset side reactors comprise ratio of benzene to olefin ranging from 5:1 to 30:1, volume ratio ranging from 2 to 16 and space velocity LHSV ranging from 6 to 9.

**32.** Process according to claim 20, **characterized in that** it the catalytic beds show molar ratio of benzene and olefin ranging from 20 to 120, volume ratio ranging from 8 to 50 and space velocity LHSV ranging from 6 to 15.

**33.** Process according to claim 20 **characterized in that** it the olefin feeding stream is comprised of alpha and inner monounsaturated olefin, linear or branched containing from 1 to 12 carbon atoms, wherein the feeding stream flow rate is in the range from 1 to 3 m³/h.

**34.** Process according to claim 20, **characterized in that** it further comprises feeding multipoints in the reactive catalytic distillation column for entry of feeding streams.

**35.** Process according to claim 20 **characterized in that** the zeolite is a dealuminated and modified zeolite USHY, preferably doped with 5 - 10% of niobium and tin.
